(19) Europäisches Patentamt
European Patent Office
Office européen des brevets

(11) EP 1 568 385 A1

(12) EUROPEAN PATENT APPLICATION

(43) Date of publication:
31.08.2005 Bulletin 2005/35

(51) Int Cl.⁷: A61L 15/60

(21) Application number: 04004132.9

(22) Date of filing: 24.02.2004

(84) Designated Contracting States:
AT BE BG CH CY CZ DE DK EE ES FI FR GB GR HU IE IT LI LU MC NL PT RO SE SI SK TR
Designated Extension States:
AL LT LV MK

(71) Applicant: THE PROCTER & GAMBLE COMPANY
Ohio 45202 (US)

(72) Inventor: Flohr, Andreas
61476 Kronberg (DE)

(74) Representative: Borbach, Markus et al
Procter & Gamble Service GmbH
65823 Schwalbach/Ts. (DE)

# (54) Superabsorbent polymers comprising direct covalent bonds between polymer chain segments

(57) The present invention relates to superabsorbent polymer particles with improved surface cross-linking and their use in absorbent articles.

Superabsorbent polymers of the invention comprise polymer chain segments, wherein at least a part of these polymer chain segments are cross-linked to each other through direct covalent bonds.

Moreover, the invention relates to a process for making these superabsorbent polymer particles.

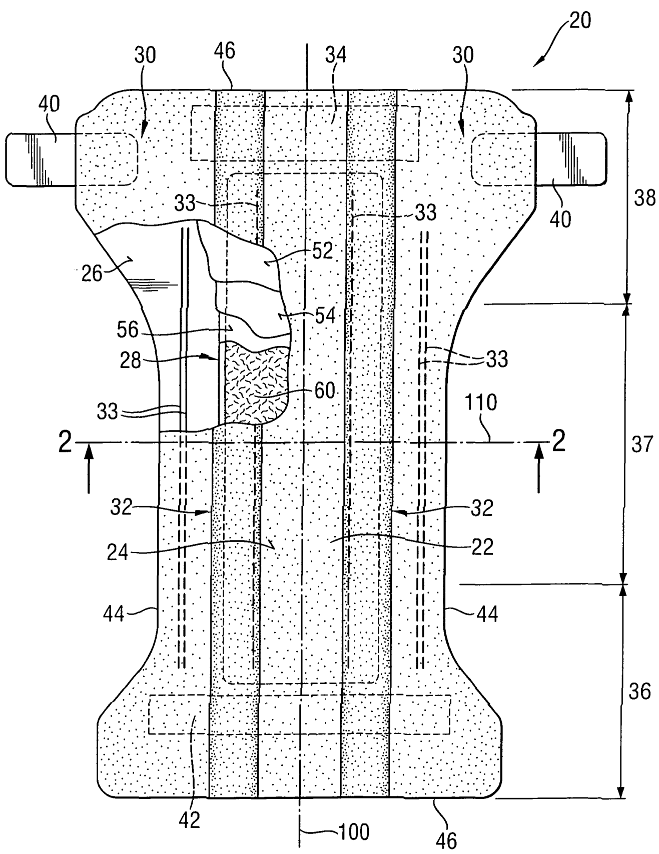


Fig. 1

EP 1 568 385 A1

## Description

### Field of the invention

The present invention relates to superabsorbent polymers comprising polymer chain segments, which are directly bound to each other through covalent bonds.

Moreover, the invention relates to a process for making these superabsorbent polymer particles and to absorbent articles comprising these superabsorbent polymers.

### Background of the invention

Superabsorbent polymers (SAPs) are well known in the art. They are commonly applied in absorbent articles, such as diapers, training pants, adult incontinence products and feminine care products to increase the absorbent capacity of such products while reducing their overall bulk. The SAPs generally are capable of absorbing and retaining amounts of aqueous fluids equivalent to many times their own weight.

Commercial production of SAPs began in Japan in 1978. The early superabsorbent was a cross-linked starch-polyacrylate. Partially neutralized polyacrylic acid eventually replaced earlier superabsorbents in the commercial production of SAPs, and is the primary polymer employed for SAPs today. They generally consist of a partially neutralized lightly cross-linked polymer network, which is hydrophilic and permits swelling of the network once submerged in water or an aqueous solution such as physiological saline. The cross-links between the polymer chains assure that the SAP does not dissolve in water. SAPs are often applied in form of small particles, such as fibers or granules.

After absorption of an aqueous solution, swollen SAP particles become very soft and deform easily. Upon deformation the void spaces between the SAP particles may be blocked, which drastically increases the flow resistance for liquids. This is generally referred to as "gel-blocking". In gel blocking situations liquid can move through the swollen SAP particles only by diffusion, which is much slower than flow in the interstices between the SAP particles.

One commonly applied way to reduce gel-blocking is to make the particles stiffer, which enables the SAP particles to retain their original shape thus creating or maintaining void spaces between the particles. A well-known method to increase stiffness is to cross-link the carboxyl groups exposed on the surface of the SAP particles. This method is commonly referred to as surface cross-linking.

European Patent EP 0 509 708 B1 refers to surface cross-linked and surfactant coated absorbent resin particles and a method of their preparation. The surface cross-linking agent in EP 0 509 708 B1 is a polyhydroxyl compound comprising at least two hydroxyl groups, which react with the carboxyl groups on the surface of the SAP particles. In EP 0 509 708 B1, surface cross-linking is carried out at temperatures of 150°C or above. The particles are preferably exposed to the elevated temperatures for at least 5 minutes but less than 60 minutes.

U.S. Patent 5,164,459 discloses another method for surface cross-linking absorbent resins, wherein the carboxyl groups of the polymer, which are comprised on the surface of the resin, react with a polyhydric alcohol. The reaction is accomplished at temperatures in the range of 90°C to 250°C.

In WO 01/89591 A2 hydroxyalkylurea is used as cross-linking agent. WO 01/89592 applies hydroxyalkylamide as cross-linking agent. In both applications, the surface cross-linking reaction is carried out at temperatures from about 90°C to about 170°C for 60 to 180 minutes.

A water-soluble peroxide radical initiator as surface cross-linking agent is known from European Patent Application EP 0 248 437 A2. An aqueous solution containing the surface cross-linking agent is applied on the surface of the polymer. The surface cross-linking reaction is achieved by heating to a temperature such that the peroxide radical initiator is decomposed while the polymer is not decomposed.

European Patent Application EP 1 199 327 A2 discloses the use of an oxetane compound and / or an imidazolidinone compound for use as surface cross-linking agent. The surface cross-linking reaction is carried out under heat, wherein the temperature is preferably in the range of 60°C to 250°C. Alternatively, the surface cross-linking reaction in EP 1 199 327 A2 is achieved by a photo-irradiation treatment, preferably using ultraviolet rays.

All processes known from the prior art result in surface cross-linked particles wherein the reaction products of the surface cross-linking molecules are built into the SAP particles. Hence, the surface cross-linked SAP particles comprise the reaction product of the cross-linking molecules.

In general, the surface cross-linking agent is applied on the surface of the SAP particles. Therefore, the reaction preferably takes place on the surface of the SAP particles, which results in improved cross-linking on the surface while not substantially affecting the core of the particles. Hence, the SAP particles become stiffer and gel-blocking is reduced.

A drawback of the commercial surface cross-linking process described above is that it takes a relatively long time, commonly at least about 30 min. However, the more time is required for the surface cross-linking process, the more surface cross-linking agent will penetrate into the SAP particles, resulting in increased cross-linking inside the

particles, which has a negative impact on the capacity of the SAP particles. Therefore, it is desirable to have short process times for surface cross-linking. Furthermore, short process times are also desirable with respect to an overall economic SAP particle manufacturing process.

Another drawback of common surface cross-linking processes is that they take place only under relatively high temperatures, often around 150°C or above. At these temperatures, not only the surface cross-linker reacts with the carboxyl groups of the polymer, but also other reactions are activated, e.g. anhydride-formation of neighboured carboxyl groups within or between the polymer chains, and dimer cleavage of acrylic acid dimers incorporated in the SAP particles. Those side reactions also affect the core, decreasing the capacity of the SAP particles. In addition, exposure to elevated temperatures can lead to colour degradation of the SAP particles. Therefore, these side reactions are generally undesirable.

SAPs known in the art are typically partially neutralized, e.g. with sodium hydroxide. However, in the processes known in the art, neutralization has to be carefully balanced with the need for surface cross-linking: The surface cross-linking agents known in the art only react with free carboxyl groups comprised by the polymer chains but they are not able to react with a neutralized carboxyl groups. Thus, the carboxyl groups can either be applied for surface cross-linking or for neutralization, but the same carboxyl group cannot be applied to fulfil both tasks. Surface cross-linking agents known in the art generally do not react with chemical groups other than carboxyl groups, e.g. they do not react with aliphatic groups.

In the process of making SAP particles, neutralization of free carboxyl groups typically comes first, before surface cross-linking takes place. Indeed, the neutralization step is often carried out in the very beginning of the process, before the monomers are polymerized and cross-linked to form the SAP. Such a process is named 'pre-neutralization process'. Alternatively, the SAP can be neutralized in the middle of polymerization or after polymerization ('post-neutralization'). Furthermore, a combination of these alternatives is also possible.

As the overall number of free carboxyl groups on the outer surface of the SAP particles is limited by the foregoing neutralization, it is very difficult to obtain particles with a high degree of surface cross-linking and hence, a high stiffness to reduce gel-blocking. Furthermore, it is very difficult to obtain SAP particles with evenly distributed surface cross-linking, as the remaining free carboxyl groups are not only few in number but generally also randomly distributed, which sometimes results in SAP particles with regions of rather dense surface cross-linking and regions of sparsely surface cross-linking.

It is therefore an objective of the present invention to provide SAP particles, which have a high degree of surface cross-linking and at the same time allow for a high degree of neutralization.

It is a further objective of the present invention to provide SAP particles with evenly distributed, homogenous surface cross-linking. Moreover, the surface comprising the surface cross-linking should be as thin as possible.

In addition, it is an even further objective of the present invention to provide SAPs and SAP particles, wherein the polymer chain segments comprised by the SAPs or SAP particles are cross-linked to each other without the need for a cross-linking molecule being built into the SAPs. This objective is especially desirable with respect to surface cross-linking, i.e. it is desirable to provide surface cross-linked SAP particles, which do not comprise the reaction product of the cross-linking molecules.

Furthermore, it is an objective of the present invention to provide a process to produce SAPs and SAP particles with the above-mentioned advantages.

It is a still further objective of the present invention to provide a process to produce SAP particles, wherein the process step of surface cross-linking can be carried out quickly to increase the efficiency of the process.

Moreover, a further objective of the present invention is to provide a process to produce SAP particles, which can be carried out at moderate temperatures in order to reduce undesired side reactions, initiated by elevated temperatures, such as anhydride-formation and dimer cleavage.

## Summary of the invention

The present invention refers to superabsorbent polymers comprising polymer chain segments. At least a part of the polymer chain segments are cross-linked to each other through covalent bonds, wherein the covalent bonds are formed directly between between polymer chain segments.

The present invention refers further to a method of making superabsorbent polymers which comprises the steps of

a) providing a polymer comprising polymer chain segments, and
b) providing a monofunctional radiation activatable radical former, and
c) exposing the polymer and the monofunctional radiation activatable radical former to electromagnetic radiation, thereby forming direct covalent bonds between the polymer chain segments.

## Brief description of the drawings

While the specification concludes with claims pointing out and distinctly claiming the present invention, it is believed the same will be better understood by the following drawings taken in conjunction with the accompanying specification wherein like components are given the same reference number.

Figure 1 is a top plan view of a disposable diaper, with the upper layers partially cut away.

Figure 2 is a cross-sectional view of the disposable diaper shown in Figure 1

Figure 3 shows the 300 MHz 1 H-NMR spectrum of di-benzoyl peroxide (BP) and benzoic acid (BA).

Figure 4 shows the $^1$H-NMR spectrum of the ether extract before photolysis, after 1 minute photolysis and after 10 minutes photolysis.

Figure 5 shows a graph which illustrates the swelling behaviour of a lightly cross-linked partially neutralized PAA in physiological saline solution before and after further cross-linking of different radical formers

## Detailed description of the invention

SAPs are available in a variety of chemical forms, including substituted and unsubstituted natural and synthetic polymers, such as carboxymethyl starch, carboxymethyl cellulose, and hydroxypropyl cellulose; nonionic types such as polyvinyl alcohol, and polyvinyl ethers; cationic types such as polyvinyl pyridine, polyvinyl morpholinione, and N,N-dimethylaminoethyl or N,N-diethylaminopropyl acrylates and methacrylates, and the respective quaternary salts thereof. Typically, SAPs useful herein have a multiplicity of anionic, functional groups, such as sulfonic acid, and more typically carboxyl groups. Examples of polymers suitable for use herein include those, which are prepared from polymerizable, unsaturated, acid-containing monomers. Thus, such monomers include the olefinically unsaturated acids and anhydrides that contain at least one carbon-to-carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually in minor amounts, in preparing SAPs. Such non-acid monomers can include, for example, the water-soluble or water-dispersible esters of the acid-containing monomers, as well as monomers that contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups, quaternary ammonium salt groups, aryl groups (e.g., phenyl groups, such as those derived from styrene monomer). These non-acid monomers are well-known materials and are described in greater detail, for example in U.S. Patent 4,076,663 and in U.S. Patent 4, 062, 817.

Olefinically unsaturated carboxylic acid and carboxylic acid anhydride monomers include the acrylic acids typified by acrylic acid itself, methacrylic acid, ethacrylic acid, α-chloroacrylic acid, a-cyanoacrylic acid, β-methylacrylic acid (crotonic acid), α-phenylacrylic acid, β-acryloxypropionic acid, sorbic acid, α-chlorosorbic acid, angelic acid, cinnamic acid, p-chlorocinnamic acid, β-sterylacrylic acid, itaconic acid, citroconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyl toluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxypropyl sulfonic acid and 2-acrylamide-2-methylpropane sulfonic acid.

Preferred SAPs useful for the present invention comprise carboxyl groups. These polymers comprise hydrolyzed starch-acrylonitrile graft copolymers, partially neutralized hydrolyzed starch-acrylonitrile graft copolymers, starch-acrylic acid graft copolymers, partially neutralized starch-acrylic acid graft copolymers, saponified vinyl acetate-acrylic ester copolymers, hydrolyzed acrylonitrile or acrylamide copolymers, slightly network crosslinked polymers of any of the foregoing copolymers, partially neutralized polyacrylic acid, and slightly network crosslinked polymers of partially neutralized polyacrylic acid, partially neutralized polymethacrylic acid, and slightly network crosslinked polymers of partially neutralized polymethacrylic acid. These polymers can be used either solely or in the form of a mixture of two or more different polymers, that when used as mixtures, individually do not have to be partially neutralized, whereas the resulting copolymer has to be. Examples of these polymer materials are disclosed in U.S. Patent 3,661,875; U.S. Patent 4,076,663; U.S. Patent 4,093,776; U.S. Patent 4,666,983; and U.S. Patent 4,734,478.

The SAPs useful for the present invention preferably comprise a homopolymer of partially neutralized α,β-unsaturated carboxylic acid or a copolymer of partially neutralized α,β-unsaturated carboxylic acid copolymerized with a monomer copolymerizable therewith.

A suitable method for polymerizing monomers is aqueous solution polymerization, which is well known in the art. An aqueous solution comprising monomers and polymerization initiator is subjected to a polymerization reaction.

The aqueous solution may comprise e.g. α,β-unsaturated carboxylic acid monomers, or may, alternatively, comprise α,β-unsaturated carboxylic acid monomers and additional monomers, which are copolymerizable with the α,β-unsaturated carboxylic acid monomers. At least the α,β-unsaturated carboxylic acid should be partially neutralized, either prior to polymerization of the monomers, during polymerization or after polymerization. In case the α,β-unsaturated carboxylic acid is partially neutralized prior to polymerization, the monomers (including α,β-unsaturated carboxylic acid monomers and possible comonomers) are at least 50 %, more preferably at least 70%, even more preferably at least 75% and even more preferably from 75% to 95% neutralized.

The monomers in aqueous solution are polymerized by standard free radical techniques, commonly by using a photoinitiator for activation, such as ultraviolet (UV) light. Alternatively, a redox initiator may be used. In this case, however, increased temperatures are necessary.

The polymer chains will preferably be lightly cross-linked to render them water-insoluble. The desired cross-linked structure may be obtained by the co-polymerization of the selected water-soluble monomer and a cross-linking agent possessing at least two polymerizable double bonds in the molecular unit. The cross-linking agent is present in an amount effective to cross-link the water-soluble polymer. The preferred amount of cross-linking agent is determined by the desired degree of absorption capacity and the desired strength to retain the absorbed fluid, that is, the desired absorption under load. Typically, the cross-linking agent is used in amounts ranging from 0.0005 to 5 parts by weight per 100 parts by weight of monomers (including α,β-unsaturated carboxylic acid monomers and possible co-monomers) used. If an amount over 5 parts by weight of cross-linking agent per 100 parts is used, the resulting polymer has a too high cross-linking density and exhibits reduced absorption capacity and increased strength to retain the absorbed fluid. If the cross-linking agent is used in an amount less than 0.0005 parts by weight per 100 parts, the polymer has a too low cross-linking density and when contacted with the fluid to be absorbed becomes rather sticky, water-soluble and exhibits a low absorption performance, particularly under load. The cross-linking agent will typically be soluble in the aqueous solution.

Alternatively to co-polymerizing the cross-linking agent with the monomers, it is also possible to cross-link the polymer chains in a separate process step after polymerization.

After polymerization, cross-linking and partial neutralization, the viscous SAPs are dehydrated (i.e. dried) to obtain dry SAPs. The dehydration step can be performed by heating the viscous SAPs to a temperature of about 120°C for about 1 or 2 hours in a forced-air oven or by heating the viscous SAPs overnight at a temperature of about 60°C.

The SAPs can be transferred into particles of numerous shapes. The term "particles" refers to granules, fibers, flakes, spheres, powders, platelets and other shapes and forms known to persons skilled in the art of SAPs. E.g. the particles can be in the form of granules or beads, having a particle size of about 10 to 1000 μm, preferably about 100 to 1000 μm. In another embodiment, the SAPs can be in the shape of fibers, i.e. elongated, acicular SAP particles. In those embodiments, the SAP fibers have a minor dimension (i.e. diameter of the fiber) of less than about 1 mm, usually less than about 500 μm, and preferably less than 250 μm down to 50 μm. The length of the fibers is preferably about 3 mm to about 100 mm. The fibers can also be in the form of a long filament that can be woven.

The present invention relates to SAPs comprising polymer chain segments, wherein at least a part of said polymer chain segments are cross-linked to each other through covalent bonds formed directly between the polymer chain segments.

A "direct covalent bond" according to the present invention means, that the polymer chain segments are bound to each other only via a covalent bond with no intermediate atoms, such as atoms comprised by a cross-linking molecule. On the contrary, known cross-linking reactions between polymer chain segments always result in covalent bonds between these polymer chain segments, wherein the reaction product of the cross-linking molecule is built in between the polymer chain segments. Thus, known cross-linking reactions do not result in a direct covalent bond but in an indirect covalent bond comprising the reaction product of the cross-linking molecule.

The method of making such SAPs can be applied on polymer chains, which have not been cross-linked to each other. Hence, the polymer chains are provided as a plurality of polymer chains, wherein the polymer chains may at least partially be branched.

Alternatively, the method can be applied for polymer chains, which have already been cross-linked by a cross-linker known in the art, comprising at least two polymerizable double bonds in the molecule unit. E.g. the method can be applied polymer chains comprised by SAP particles, e.g. for surface cross-linking. However, the direct covalent bonds between polymer chain segments according to the present invention are not intended to bond different SAP particles to each other. Thus, the method of the present invention, when applied on SAP particles, does not lead to any appreciable inter-particulate direct covalent bonds between different SAP particles but only results in intra-particulate direct covalent bonds within an SAP particle. If present, such interparticulate direct covalent bonds would hence require additional inter-particulate cross-linking materials, such as cross-linking molecules.

For applications, wherein the polymer chains have already been cross-linked and are thus provided in form of a network, the term "polymer chain segment" refers to the part of the polymer chains between two neighbouring, existing cross-links or to the part of the polymer chains between sites, where the polymer chain is branched.

However, if the polymer chains have not been pre-cross-linked at all prior to subjecting them to the cross-linking process of the present invention, the term "polymer chain segments" refers to a complete individual polymer chain.

In a preferred embodiment of the present invention, the polymer chain segment comprises polycarboxylic acid units. According to the present invention, the term "polycarboxylic acid unit" refers to a unit consisting of at least two carboxylic acid monomer units, which have been polymerized to each other and which are part of a larger polymer. The term "carboxylic acid monomer units" refers to the reaction product of the carboxylic acid monomer after the polymerization reaction and thus refers to the carboxylic acid monomer built into the polymer. In a preferred embodiment of the present invention, the polycarboxylic acid units consist of polyacrylic acid units or of polymethacylic acid units. A polyacrylic acid unit consists of at least two acrylic acid monomer units, which have been polymerized to each other. A polymethacrylic acid unit consists of at least two methacrylic acid monomer units, which have been polymerized to each other. Alternatively, the carboxylic acid unit may also consist of acrylic acid monomers units and methacylic acid monomers units, which have been copolymerized.

According to the present invention, the polycarboxylic acid units are at least partially neutralized, i.e. at least a part of the carboxylic acid units are neutralized.

Additional to the polycarboxylic acid units, the polymer chain segments may further comprise other units, such as polystyrene units. According to the present invention, the term "polystyrene unit" refers to a unit consisting of at least two styrene monomer units, which have been polymerized to each other and which are part of a larger polymer. The term "styrene monomer units" refers to the reaction product of the styrene monomer after the polymerization reaction and thus refers to the styrene monomer built into the polymer.

The polymer chain segment comprising e.g. polycarboxylic acid units in combination with other polymer units, such as polystyrene units is referred to as a "block polymer chain segment".

Most preferred polymers for use herein are slightly network crosslinked polymers of partially neutralized polyacrylic acids, slightly network crosslinked polymers of partially neutralized polymethacrylic acids, their copolymers and starch derivatives thereof. Most preferably, SAPs comprise partially neutralized, slightly network crosslinked, polyacrylic acid (i.e. poly (sodium acrylate/acrylic acid)). Preferably, the SAPs are at least 50%, more preferably at least 70%, even more preferably at least 75% and even more preferably from 75% to 95% neutralized. Network cross-linking renders the polymer substantially water-insoluble and, in part, determines the absorptive capacity of the hydrogel-forming absorbent polymers. Processes for network cross linking these polymers and typical network cross linking agents are described in greater detail in U.S. Patent 4,076,663.

In the most preferred embodiment of the present invention, the method of directly bonding polymer chain segments to each other by a covalent bond is applied for surface cross-linking SAP particles instead of or additional to conventional surface cross-linking.

It has been found that a radical former comprising at least one radiation activatable group, upon activation by electromagnetic radiation can abstract a proton from a polymer chain segment. Two of those radicals induced in the polymer chain segments can combine to form a direct covalent bond between polymer chain segments. According to the present invention, a radical former comprising only one radiation activatable group is monofunctional.

Preferred monofunctional radical formers according to the present invention include di-benzoyl peroxide, 3-benzoyl benzoic acid, 1,3-diibenzoyl propane and trifluoromethyl phenylketone.

In the following, the reactions potentially taking place when applying a monofunctional radical former to polymer chain segments is exemplarily depicted for di-benzoyl peroxide as radical former and for polymer chain segments comprising polyacrylic acid units (PAA):

1) Upon UV initiation, the di-benzoyl peroxide forms benzoyl benzoic acid radicals according to Formula 1a.

<u>Formula 1a</u>:

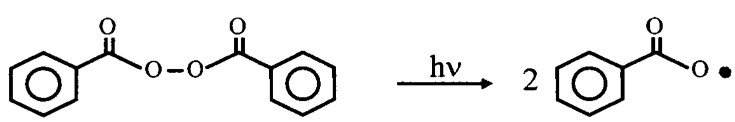


The benzoic acid radical may theoretically de-carboxylate to form benzene radicals as depicted in Formula 1b.

Formula 1b:

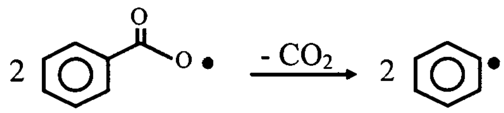


After this initial reaction, all of the following reactions 2) to 9) may theoretically take place:

2) The benzoyl benzoic acid radicals can recombine to again form di-benzoyl peroxide as depicted in Formula 2.

Formula 2:

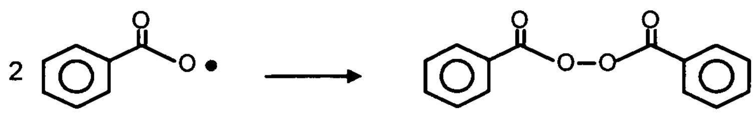

3) Alternatively to the reaction of Formula 2, the benzoyl benzoic acid radical can react with the benzene radical to form the corresponding esther, according to Formula 3.

Formula 3:

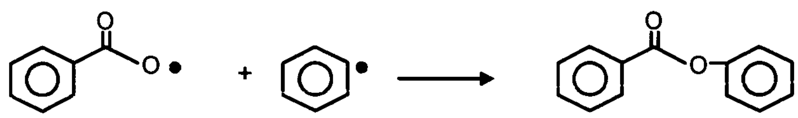

4) Also, a recombination of two benzene radicals to form bi-phenyl as in Formula 4 is theoretically possible.

Formula 4:

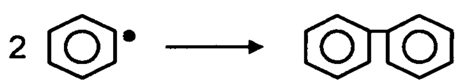

5) As a further alternative, the benzoyl benzoic acid radical can react with the PAA to form benzoic acid and a PAA-radical, according to Formula 5.

Formula 5:

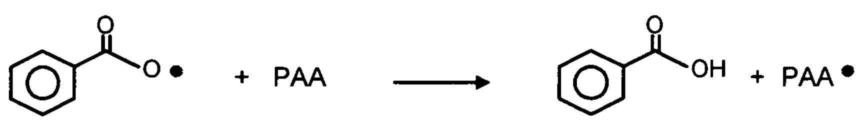

The benzoic acid may be subjected to an oxidative reaction to regenerate the radical former di-benzoyl peroxide.

6) Furthermore, benzene radicals, if formed, could also react with the PAA according to Formula 6 to form benzene and a PAA-radical.

Formula 6:

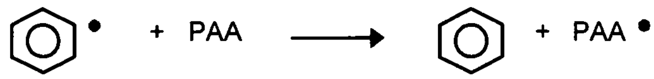

If PAA-radicals according to Formula 5 and 6 have been formed, the following reactions comprising PAA-radicals may principally take place:

7) The PAA-radical can, in turn, react again with the benzoyl benzoic acid radical, whereby the benzoyl benzoic acid radical is attached to the PAA polymer chain segment as depicted in Formula 7.

Formula 7:

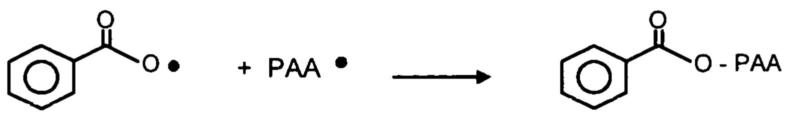

8) Alternatively, the PAA-radical can react with the benzene radical to attach the benzene radical to the polymer chain as depicted in Formula 8.

Formula 8:

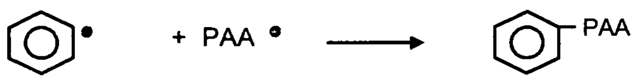

9) As a second alternative, two PAA-radicals can recombine, according to Formula 9.

Formula 9:

$$2\ PAA \bullet \rightarrow PAA - PAA$$

The reaction according to Formula 9 results in polymer chain segments, which are directly covalently bound to each other.

To determine, which of the above reactions have actually taken place, the reaction samples are extracted with ether after UV initiation, and the extracts are analysed via $^{1}H$-NMR and $^{19}F$-NMR (for example in case tri-fluoromethyl phenylketone is used as radical former). Figure 3 depicts the 300 MHz $^{1}H$-NMR spectra of di-benzoyl peroxide (BP) and benzoic acid (BA).

In the above-mentioned example where the radical former is di-benzoyl peroxide, the $^{1}H$-NMR spectra of the ether extract reveals the presence of benzoic acid, according to Figure 4. Benzoic acid is only formed in the reaction according to Formula 5. Moreover, the reaction according to Formula 5 can only take place if the benzoyl benzoic acid radicals are generated according to Formula 1a. Hence, the ether extract showed that the reactions as depicted in Formula 1 a and 5 actually take place. Thus, PAA-radicals according to Formula 5 must indeed have been formed.

Surprisingly, the esther according to Formula 3 could not be detected in the ether extract. Moreover, neither the bi-phenyl as a reaction product of the reaction according to Formula 4, nor the reaction products of Formula 7 or Formula 8 were found in the ether extract. Thus, none of the reactions involving benzene radicals took place. Consequently, no benzene radicals according to Formula 1 b are formed.

Accordingly, as the $^{1}H$-NMR spectra of Figure 4 derived from the ether extracts shows that out of the reactions depicted in Formulae 1 to 8, only the reactions of Formula 1a, Formula 5 and possibly Formula 2 (recombination of the radical former) actually take place, the reaction of Formula 9 also takes place: The PAA-radicals, which are formed as depicted in Formula 5 neither react with the benzoyl benzoic acid radicals according to Formula 7 nor do they react with the benzene radical according to Formula 8. However, as free radicals are not stable and no other reaction partner

has been available, the PAA-radicals must have recombined according to the reaction depicted in Formula 9. Thus, it can be concluded from analysis of the ether extracts that indeed a direct covalent bond between different polymer chain segments has been built.

This direct bond cannot comprise the reaction product of the radical former because the radical former only comprises one radiation activatable group and is thus not able to form a cross-link upon UV radiation as this would require at least two radiation activatable groups. Consequently, when applying di-benzoyl peroxide as radical former to polymer chain segments comprising polyacrylic acid (PAA) and subsequently UV initiating the di-benzoyl peroxide, the following reaction takes place:

Formula 10:

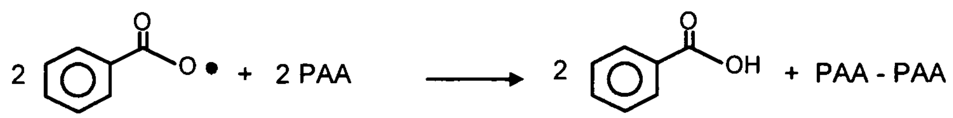

The Graph in Figure 5 illustrates the swelling behaviour of a lightly cross-linked partially neutralized PAA in physiological (0.9 molar) saline (NaCl) solution before (Reference; Sample 1) and after further cross-linking of different radical formers.

In each case, 200 mg of Reference PAA was mixed with 20 mg of the respective radical former either in the dry state (Samples 5 and 6) or dissolved/suspended in 1.5 ml of water (Samples 2 to 4, 7 and 8) and photolysis was carried out for either 10 or 60 minutes according to the photolysis method described below.

Prior to the addition of the 0.9 molar saline solution, sample 2 contains the reaction product of lightly cross-linked partially neutralized PAA with an aqueous solution/slurry of trifluoromethyl phenylketone after photolysis; sample 3 contains the reaction product of lightly cross-linked partially neutralized PAA with an aqueous solution/slurry of the sodium salt of 3-benzoyl benzoic acid after photolysis; sample 4 contains the reaction product of lightly cross-linked partially neutralized PAA with an aqueous solution/slurry of di-benzoyl peroxide after photolysis; samples 5 and 6 contain the reaction product of lightly cross-linked partially neutralized PAA after dry addition of the sodium salt of 3-benzoyl benzoic acid and subsequent photolysis for 10 min and 60 min, respectively, whereas samples 7 and 8 contain the reaction product of lightly cross-linked partially neutralized PAA with an aqueous solution/slurry of the sodium salt of 3-benzoyl benzoic acid and subsequent photolysis for 10 min and 60 min, respectively.

Figure 5 clearly shows that after photolysis of the Reference material in the presence of the respective radical former, the swelling capacity of such reaction product is dramatically reduced, swelling is increasingly restricted, hence additional cross-links of neighbouring polymer chain segments according to Formula 9 are formed.

Figure 5 also exhibits that for samples 7 and 8 the reaction efficiencies are increased once photolysis is carried out in aqueous solution/slurry rather than in the dry state when compared to samples 5 and 6.

In the example depicted in Figure 5, 10% of radical former by weight of the polymer is added to the polymer. However, in most embodiments of the present invention, lower degrees of direct covalent bonds than those shown in Figure 5 will be preferred. Thus, a preferred range for the addition of the radical former to the polymer is between 0.01 % and 10% by weight of the polymer, more preferred between 0.01% and 5%, even more preferred between 0.01 and 2% and most preferred between 0.01% and 1%.

In covalent cross-linking of polymer chains known in the art, a cross-linker has been required and the reaction product of the cross-linker molecule has been built into the network comprising the polymer chain segments. Thus, the polymer chain segments have not been bound to each other directly but via the reaction product of the surface cross-linker.

Moreover, the cross-linkers known in the art had to be at least bi-functional to be able to covalently cross-link different polymer chain segments to each other: Examples are the thermally activated surface cross-linkers such as di- or polyhydric alcohols, or derivatives thereof, as discussed above.

Contrary thereto, the radical formers of the present invention are mono-functional. The radical formers are capable to form radicals upon exposure to electromagnetic radiation. Electron beams as well as UV-light can produce suitable electromagnetic radiation. Preferably, according to the present invention UV-light is used with a wavelength of 220-380 nm, most preferably with a wave-length between 330-365 nm. The UV-light may be used in combination with an electron-beam, and also in combination with an IR-light. In case of combination of UV-irradiation with other electromagnetic radiation is used, it is not critical if the application of the UV-light takes place simultaneously with the other electromagnetic irradiation (i.e. electron-beam or IR-light), or if irradiation is done in a series of different irradiation steps. For radical formers, which require a relative high amount of activation energy, activation with electron beams may be necessary.

The UV radiation according to the present invention is preferably carried out at intensities between 10 and 300 W/cm, more preferably between 10 W/cm and 200 W/cm, even more preferred between 10 W/cm and 150 W/cm and most preferred between 10 W/cm and 120 W/cm. Irradiation time is preferably between 0.1 sec. and 30 min., more preferably between 0.1 sec. and 15 min, even more preferably between 0.1 sec. and 5 min and most preferably between 0.1 sec. and 2 min. Commercially available mercury pressure UV-lamps can be used.

Upon electromagnetic radiation, such as UV radiation, the radical formers form free radicals upon. The highly reactive free radicals formed thereby are able to react with polymer chain segments comprised by the superabsorbent polymer. When a free radical formed from the radical former reacts with a polymer chain segment, the polymer chain segment forms a "polymer chain segment radical". It is believed that reaction within the polymer chain segment takes place on an aliphatic group (C-H group) comprised by the polymer chain segment. Alternatively, the reaction may also take place on those carboxylic groups comprised by the polymer chain segment, which have not been neutralized. A further alternative is that the reaction takes place on another functional group comprised by the polymer chain segment if the functional group comprises a Proton that can be abstracted. Examples of such functional groups are sulfonic acid, carboxylic acid or sulfonic acid esters, hydroxyl groups, amide-groups, amino groups, nitrile groups, quaternary ammonium salt groups, aryl groups (e.g., phenyl groups, such as those derived from styrene monomer). When two such polymer chain segment radicals react with each other, a direct covalent bond between the polymer chain segments is formed.

It is believed that the reaction which leads to direct covalent bonds between polymer chain segments preferably takes place on molecules comprised by the polymer backbone.

Preferred radical formers according to the present invention have a molecular weight of at least Mw = 25 g/mol, more preferred at least Mw = 60 g/mol, still more preferred at least Mw = 120 g/mol, even more preferred at least Mw = 180 g/mol and most preferred at least Mw = 240 g/mol. Radical formers having a relatively high molecular weight often tend to form more stable radicals, as the charge of the radical can be distributed better within the radical. Hence, the radical is more likely to reach a polymer chain segment within the reaction solution and are able to react with the polymer chain segment to form a "polymer chain segment radical". If the radical were very unstable, it would more likely react to recombine to the radical former as depicted e.g. in Formula 2.

Furthermore, preferred radical formers according to the present invention will comprise aromatic groups, such as arenes. This also leads to more stable radicals as the charge can be distributed throughout the aromatic group.

According to the present invention the dehydrated SAP particles may undergo a surface cross-linking process step. The term "surface" describes the outer-facing boundaries of the particle. For porous SAP particles, exposed internal surfaces may also belong to the surface. The term "surface cross-linked SAP particle" refers to an SAP particle having its polymer chain segments present in the vicinity of the particle surface cross-linked to each other. It is known in the art to surface cross-link the polymer chain segments present in the vicinity of the particle surface by a compound referred to as surface cross-linker. The surface cross-linker is applied to the surface of the particle. In a surface cross-linked SAP particle the level of cross-links in the vicinity of the surface of the SAP particle is generally higher than the level of cross-links in the interior of the SAP particle.

Commonly applied surface cross-linkers are thermally activated surface cross-linkers. The term "thermally activated surface cross-linkers" refers to surface cross-linkers, which only react upon exposure to increased temperatures, typically around 150°C. Thermally activated surface cross-linkers known in the prior art are e.g. di- or polyfunctional agents that are capable of building additional cross-links between the polymer chains of the SAPs. Other thermally activated surface cross-linkers include, e.g., di- or polyhydric alcohols, or derivatives thereof, capable of forming di- or polyhydric alcohols. Representatives of such agents are alkylene carbonates, ketales, and di- or polyglycidlyethers. Moreover, (poly)glycidyl ethers, haloepoxy compounds, polyaldehydes, polyoles and polyamines are also well known thermally activated surface cross-linkers. The cross-linking is based on a reaction between the functional groups comprised by the polymer, for example, an esterification reaction between a carboxyl group (comprised by the polymer) and a hydroxyl group (comprised by the surface cross-linker). As typically a relatively big part of the carboxyl groups of the polymer chain segments is neutralized prior to the polymerization step, commonly only few carboxyl groups are available for this surface cross-linking process known in the art. E.g. in a 70% percent neutralized polymer only 3 out of 10 carboxylic groups are available for covalent surface cross-linking.

According to the present invention, surface cross-linking does not have to comprise a surface cross-linker, the reaction product of which will be built into the SAP particle after surface cross-linking. On the contrary, according to the present invention, it is possible to surface cross-link the polymer chain segments by directly bonding the polymer chain segments to each other through a covalent bond. The radical former, which initiates the reaction, does not get built in the SAP particle. Optionally, the final reaction product of the radical former can be regenerated after surface cross-linking and hence, after regeneration, can be used again for surface cross-linking. No additional monomers, such as carboxylic acids or styrenes, are required if the radical former is used for surface cross-linking of SAP particles.

If the radical former is used for surface cross-linking of SAP particles, the direct covalent bonds between polymer chain segments on the surface of the SAP particles are formed intra-particulate. They are not intended to form

inter-particle bonds.

Further, if the radical former is used for surface cross-linking of SAP particles, the radical former may be sprayed onto the SAP particles by means of a fluidized-bed spraying chamber. Simultaneously IR-irradiation may be applied to accomplish drying and simultaneously UV-light may be applied to accomplish cross-linking in the fluidized-bed.

However, in certain cases drying and cross-linking may take place in two steps in series, which could be carried out in any order. Instead or in combination with IR-light, any conventional drying equipment can be used in the drying step. However, in certain embodiments of the present invention little or no drying is required, e.g. in cases, where only small amounts of surface cross-linkers are applied dissolved in small amounts of solution.

Prior art surface cross-linking has been restricted to carboxylic groups comprised by the polymer chain segments exposed on the surface of the SAP particle. Advantageously, the cross-linking process of the present invention is not restricted to the carboxyl groups but also comprises numerous other functional groups and aliphatic groups within the polymer chains of the SAP. Hence, according to the present invention the number of available reaction sites for the surface cross-linking process of the SAP particles is strongly increased. Therefore, it is possible to achieve a far more homogenous, uniform surface cross-linking compared to the surface cross-linking known from the art. Furthermore, it is possible to surface cross-link the SAP to a higher degree than the SAP known from the prior art. This enables to make the SAP particles much stiffer, thus, to more effectively inhibit the gel-blocking effect at any given degree of neutralization.

Surface cross-linking of SAP particles mainly takes place on the surface of the SAP particles. That means that mainly polymer chain segments, which are exposed in the vicinity of the surface of the SAP particles, undergo a cross-linking process, leading to SAP particles with a high degree of cross-linking on their surface while not substantially affecting the inner core of the SAP particles. Hence, the covalent bonds formed directly between said polymer chain segments are formed mainly on the surface of said superabsorbent particles whereas said core is substantially free of said covalent bonds.

The UV radiation for the surface cross-linking is preferably carried out at intensities between 10 and 300 W/cm, more preferably between 10 W/cm and 200 W/cm, even more preferred between 10 W/cm and 150 W/cm and most preferred between 10 W/cm and 120 W/cm. Irradiation time is preferably between 0.1 sec. and 30 min., more preferably between 0.1 sec. and 15 min, even more preferably between 0.1 sec. and 5 min and most preferably between 0.1 sec. and 2 min. Commercially available mercury pressure UV-lamps can be used.

Compared to the surface cross-linking known from the prior art, the surface cross-linking according to the present invention is much quicker. Prior art surface cross-linking reactions carried out under increased temperatures commonly take up to 45 minutes. This time consuming process step renders the manufacturing process of SAP particles less economic than desirable. On the contrary, the cross-linking process according to the present invention can be carried out very quickly and hence, strongly adds to a much more efficient and economic overall manufacturing process.

Furthermore, as the surface cross-linking reaction proceeds quickly, the surface cross-linking molecules applied on the surface of the SAP particles have less time to penetrate inside the SAP particles. As a result, the surface cross-linking process is mainly restricted to the surface of the SAP particles and avoids undesired further cross-linking reactions inside the SAP particles.

Another advantage of the present invention refers to the neutralization step. $\alpha,\beta$-unsaturated carboxylic acid monomers are often neutralized prior to the polymerization step (pre-neutralization). Compounds, which are useful to neutralize the acid groups of the monomers, are typically those, which will sufficiently neutralize the acid groups without having a detrimental effect on the polymerization process. Such compounds include alkali metal hydroxides, alkali metal carbonates and bicarbonates. Preferably, the material used for neutralization of the monomers is sodium or potassium hydroxide or carbonate. The neutralizing compound is preferably added to an aqueous solution comprising the $\alpha,\beta$-unsaturated carboxylic acid monomers (pre-neutralization). As a result, the carboxyl groups comprised by the $\alpha,\beta$-unsaturated carboxylic acid monomers are at least partially neutralized. Consequently, -after the polymerization step- also the carboxyl groups comprised by the $\alpha,\beta$-unsaturated carboxylic acid of the polymer are at least partially neutralized. In case sodium hydroxide is used, neutralization results in sodium acrylate, which dissociates in water into negatively charged acrylate monomers and positively charged sodium ions.

If the final SAP particles are in the swollen state, after they absorb aqueous solution, the sodium ions are freely movable within the SAP particles. In absorbent articles, such as diapers or training pants, the SAP particles typically absorb urine. Compared to distilled water, urine comprises a relatively high amount of salt, which at least partly is present in dissociated form. The dissociated salts comprised by the urine make absorption of liquid into the SAP particles more difficult, as the liquid has to be absorbed against an osmotic pressure caused by the ions of the dissociated salts. The freely movable sodium ions within the SAP particles strongly facilitate the absorption of liquid into the particles, because they reduce the osmotic pressure. Therefore, a high degree of neutralization can largely increase the capacity of the SAP particles and the speed of liquid absorption.

The surface cross-linkers known in the art react with the carboxyl groups of the polymer. Hence, the degree

of neutralization has to be balanced with the need to surface cross-link, because both process steps make use of the carboxyl groups.

According to the present invention, the surface cross-linking reaction using radical formers and forming direct covalent bonds between polymer chain segments is not restricted to carboxyl groups but further comprises other groups within the polymer chain segment such as aliphatic groups. Therefore, it is possible to neutralize the monomers to a larger degree without significantly diminishing the possibility of later surface cross-linking.

According to the present invention, the carboxyl groups comprised by the $\alpha,\beta$-unsaturated carboxylic acid monomers are preferably at least 50 %, more preferably at least 70%, even more preferably at least 75% and even more preferably between 75% and 95% neutralized. Hence, also the carboxyl groups comprised by the $\alpha,\beta$-unsaturated carboxylic acid of the polymer are at least 50 %, more preferably at least 70%, even more preferably at least 75% and even more preferably between 75% and 95% neutralized.

A still further advantage of the present invention is the reduction of undesired side-reactions during the surface cross-linking process. Surface cross-linking known from the prior art requires increased temperatures, commonly around or above 150°. At these temperatures, not only the surface cross-linking reaction is achieved, but also a number of other reactions take place, e.g. anhydrate-formation within the polymer or dimer cleavage of dimers previously formed by the acrylic acid monomers. These side-reactions are highly undesired, because they result in SAP particles with decreases capacity.

As the surface cross-linking process according to the present invention does not necessarily need increased temperatures but can also be carried out at moderate temperatures using electromagnetic radiation, such as UV radiation, those side-reactions are substantially eliminated. According to the present invention, the surface cross-linking reaction using radical formers and electromagnetic radiation can preferably be accomplished at temperatures of less than 100°C, preferably at temperatures less than 80°C, more preferably at temperatures less than 50°C, even more preferably at temperatures less than 40°C, most preferably at temperatures between 20°C and 40°C.

At elevated temperatures around or above 150°C commonly applied in the surface cross-linking process known from the prior art, the SAP particles sometimes change their colour from white to yellowish. As according to the surface cross-linking process of the present invention, it is possible to carry out the surface cross-linking process under moderate temperatures, the problem of colour degradation of the SAP particles is strongly reduced.

According to the present invention, one radical former can be selected or, alternatively, two or more different radical formers can be applied.

As a further alternative, one or more radical former can be applied together with one or more thermally activated surface cross-linkers, e.g. 1,4-butandiol. In this embodiment, the SAP particles have to comprise carboxyl groups wherein at least some of the carboxyl groups are at least partially exposed on the outer surface of the SAP particles and wherein the thermally activated surface cross-linker is covalently bound to at least a part of the carboxyl groups at least partially exposed on the surface of said superabsorbent polymer particles.

In case a radical former is used together with a thermally activated surface cross-linker, both UV radiation and increased temperatures (above 140°C) are necessary for the surface cross-linking process.

The radical former is preferably used in a liquid solution, more preferably in an aqueous solution.

To obtain SAP particles with evenly distributed surface cross-linking, the radical former has to be distributed evenly on the SAP particle prior to or during UV radiation. Therefore, the radical former is preferably applied by spraying onto the SAP particles.

The water-absorbing resin provided for the process is preferably dry SAP particles. Alternatively, the water-absorbing resin is pre-swollen SAP particles. The term "dry SAP particles" refers to SAPs containing from no water up to an amount of less than 5 % by weight of water (wherein 100% by weight is the amount of SAP including the water). The term "pre-swollen SAP particles" refers to SAPs containing from 5% by weight of water up to an amount of 80% by weight of water, preferably up to an amount of 50% by weight of water, more preferably up to an amount of 30% by weight of water.

However, the present invention is not restricted to surface cross-linking of SAP particles. It is also possible to directly covalently cross-link polymer chain segments well before the SAP particles have been formed. For example, the radical former can be applied to polymer chains formed from polymerization reaction of the respective monomers before the polymer chains have been cross-linked to each other to form a network. In this embodiment, the cross-linking with the radical former may replace the cross-linking processes known in the art.

Alternatively, the cross-linking according to the present invention can be carried out in addition to known cross-linking process, either prior to the known processes, simultaneously or afterwards.

In these embodiments the radical former is not applied to SAPs, which have been formed into particles. Consequently, if the polymer is transformed into SAP particles, the direct covalent cross-links between the polymer chain segments is not restricted mainly to the surface of the SAP particles, but the direct covalent bonds between polymer chain segments will be present throughout the SAP particles, possibly the direct covalent bonds will distributed homogeneously throughout the SAP particles.

Alternatively, the direct covalent bonds between polymer chain segments will be distributed in-homogeneously throughout the SAP particle: For example, it is possible to mix different polymers comprising different polymer chain segments. In this case, the different polymer chains may be cross-linked (directly or indirectly by a process known in the art) to a different degree or polymers chains in certain regions of the SAP particles may not be cross-linked at all.
It is also possible to mix different polymers for forming the SAP particles comprising different polymer chain segments. In this case, the different polymers may comprise mixtures of different homopolymers, copolymers and/or block polymers.
However, all such SAP particles comprising direct covalent bonds throughout the SAP particles may undergo surface cross-linking. In this instance, the surface cross-linking may be achieved by subjecting the SAP particles to the radical former of the present invention, by subjecting them to a surface cross-linking process known in the art or by a combination of both.

Absorbent articles

The SAP particles of the present invention are preferably applied in absorbent articles. As used herein, absorbent article refers to devices that absorb and contain liquid, and more specifically, refers to devices that are placed against or in proximity to the body of the wearer to absorb and contain the various exudates discharged from the body. Absorbent articles include but are not limited to diapers, adult incontinent briefs, diaper holders and liners, sanitary napkins and the like.
Preferred absorbent articles of the present invention are diapers. As used herein, "diaper" refers to an absorbent article generally worn by infants and incontinent persons about the lower torso.
"Disposable" is used herein to describe articles that are generally not intended to be laundered or otherwise restored or reused i.e., they are intended to be discarded after a single use and, preferably, to be recycled, composted or otherwise disposed of in an environmentally compatible manner.
Figure 1 is a plan view of a diaper 20 as a preferred embodiment of an absorbent article according to the present invention. The diaper is shown in its flat out, uncontracted state (i.e., without elastic induced contraction). Portions of the structure are cut away to more clearly show the underlying structure of the diaper 20. The portion of the diaper 20 that contacts a wearer is facing the viewer. The chassis 22 of the diaper 20 in Figure 1 comprises the main body of the diaper 20. The chassis 22 comprises an outer covering including a liquid pervious topsheet 24 and/or a liquid impervious backsheet 26. The chassis 22 may also include most or all of the absorbent core 28 encased between the topsheet 24 and the backsheet 26. The chassis 22 preferably further includes side panels 30, leg cuffs 32 with elastic members 33 and a waist feature 34. The leg cuffs 32 and the waist feature 34 typically comprise elastic members. One end portion of the diaper is configured as the front waist region 36 of the diaper 20. The opposite end portion is configured as the rear waist region 38 of the diaper 20. The intermediate portion of the diaper is configured as the crotch region 37, which extends longitudinally between the front and rear waist regions. The crotch region 37 is that portion of the diaper 20 which, when the diaper is worn, is generally positioned between the wearer's legs.
The waist regions 36 and 38 may include a fastening system comprising fastening members 40 preferably attached to the rear waist region 38 and a landing zone 42 attached to the front waist region 36.
The diaper 20 has a longitudinal axis 100 and a transverse axis 110. The periphery of the diaper 20 is defined by the outer edges of the diaper 20 in which the longitudinal edges 44 run generally parallel to the longitudinal axis 100 of the diaper 20 and the end edges 46 run generally parallel to the transverse axis 110 of the diaper 20.
The diaper may also include other features as are known in the art including front and rear ear panels, waist cap features, elastics and the like to provide better fit, containment and aesthetic characteristics.
The absorbent core 28 may comprise any absorbent material that is generally compressible, conformable, non-irritating to the wearer's skin, and capable of absorbing and retaining liquids such as urine and other certain body exudates. The absorbent core 28 may comprise a wide variety of liquid-absorbent materials commonly used in disposable diapers and other absorbent articles such as comminuted wood pulp, which is generally referred to as air felt. Examples of other suitable absorbent materials include creped cellulose wadding; melt blown polymers, including coform; chemically stiffened, modified or cross-linked cellulosic fibers; tissue, including tissue wraps and tissue laminates, absorbent foams, absorbent sponges, absorbent gelling materials, or any other known absorbent material or combinations of materials. The absorbent core may further comprise minor amounts (typically less than 10%) of non-liquid absorbent materials, such as adhesives, waxes, oils and the like.
Furthermore, the SAP particles of the present invention can be applied as absorbent materials. The SAP particles of the present invention preferably are present in amounts of at least 50% by weight of the whole absorbent core, more preferably at lest 60%, even more preferably at least 75% and still even more preferably at least 90% by weight of the whole absorbent core.
Figure 2 shows a cross-sectional view of Figure 1 taken in the transverse axis 110. In Figure 2 illustrates a preferred embodiment of the different zones comprised by the absorbent cores. In Figure 2, the fluid acquisition zone

50 comprises an upper acquisition layer 52 and a lower acquisition layer 54, while the fluid storage zone underneath the fluid acquisition zone comprises a storage layer 60, which is wrapped by an upper core wrap layer 56 and a lower core wrap layer 58.

In one preferred embodiment the upper acquisition layer comprises a nonwoven fabric whereas the lower acquisition layer preferably comprises a mixture of chemically stiffened, twisted and curled fibers, high surface area fibers and thermoplastic binding fibers. In another preferred embodiment both acquisition layers are provided from a non-woven material, which is preferably hydrophilic. The acquisition layer preferably is in direct contact with the storage layer.

In a preferred embodiment the core wrap material comprises a top layer and a bottom layer, which layers may be sealed together along their edges, e.g. by adhesive. The top layer and the bottom layer can be provided from a non-woven material. The top layer and the bottom layer may be provided from two or more separate sheets of materials or they may be alternatively provided from a unitary sheet of material. Such a unitary sheet of material may be wrapped around the storage layer, e.g. in a C-fold.

The storage layer the present invention typically comprises SAP particles mixed with fibrous materials. Other materials as suitable for the absorbent core may also be comprised.

Methods

Ether Extraction:

After photolysis, the reaction products are subjected to an ether extraction. 10 ml of commercially available, dry (≤ 0.005% water) diethyl ether is added to the reaction product after photolysis, these samples are magnetically stirred for 10 min, then centrifuged for 5 minutes at room temperature. The ether phase is removed with an Eppendorf pipette for further analysis, e.g. for NMR analysis.

NMR Spectroscopy:

For Nuclear Magnetic Resonance Spectroscopy, NMR, either deuterated trichlormethane, $CDCl_3$, or deuterated Methylenenchloride, $CD_2Cl_2$ is used as solvent for the ether extracts. For example a Bruker Avance 300 MHz NMR spectrometer with 7.05 T Oxford super conducting magnet (54 mm bore) and gradient shimming, controlled using a PC/NT Pentium III 750MHz computer, can be used. For example a 5mm four-nucleus probe observable for $^1H$, $^{13}C$, $^{19}F$ and $^{31}P$ with z-gradient, variable temperature capability (-150° C to +200° C) and boron-free glass insert can be used. Respective $^1H$, $^{13}C$, $^{19}F$ spectra are acquired by standard high-resolution (solution) NMR techniques well know to the person skilled in the art.

Photolysis:

200 mg of PAA is mixed with 20 mg of the respective radical former either in the dry state or solved/suspended in 1.5 ml of water. Photolysis is carried out for either 10 or 60 minutes with a 450 W medium pressure Hg lamp as UV source using a pyrex filter to cut out light below a wavelength of 300 nm. Such lamp mostly generates light at a wavelength of 365 nm. All samples are degassed prior to photolysis by either pumping to $10^{-5}$ torr or three freeze-pumping-thaw cycles. For improved visualization, the saline solution can be stained with the cationic dye Methylene Blue.

## Claims

1. Superabsorbent polymer comprising polymer chain segments, wherein at least a part of said polymer chain segments are cross-linked to each other through covalent bonds, **characterized in that** said covalent bonds are formed directly between said polymer chain segments.

2. Superabsorbent polymer according to claim 1, wherein said polymer chain segments comprise polycarboxylic acid units and wherein said covalent bonds are formed directly between said polycarboxylic acid units of said polymer chain segments.

3. Superabsorbent polymer according to claim 2, wherein said polycarboxylic acrylic acid units comprise polyacrylic acid units, polymethacrylic acid units or both.

4. Superabsorbent polymer according to claim 2 or 3, wherein said polycarboxylic acid units are at least partially

neutralized.

**5.** Superabsorbent polymer according to any of the preceding claims, wherein said polymer chain segments are block copolymer segments.

**6.** Superabsorbent polymer according to any of the preceding claims, wherein said superabsorbent polymer is formed into a superabsorbent polymer particles and wherein the direct covalent bonds are solely formed intraparticulate.

**7.** Superabsorbent polymer according to any of the preceding claims, wherein said superabsorbent polymer is formed into a superabsorbent polymer particle comprising a surface and a core and wherein said covalent bonds formed directly between said polymer chain segments are formed on the surface of said superabsorbent particles whereas said core is substantially free of said covalent bonds.

**8.** Superabsorbent polymer according to claim 7, wherein said polymer chain segments comprised by said surface are further cross-linked to each other through thermally activated cross-linkers.

**9.** A method of making superabsorbent polymers which comprises the steps of

a) providing a polymer comprising polymer chain segments, and
b) providing a mono-functional radiation activatable radical former, and
c) exposing said polymer and said mono-functional radiation activatable radical former to electromagnetic radiation, thereby forming direct covalent bonds between said polymer chain segments.

**10.** The method according to claim 9, wherein said radiation activatable radical former has a molecular weight of at least Mw = 25 g/mol, more preferred at least Mw = 60 g/mol.

**11.** The method according to claim 9 or 10, wherein said radiation activatable radical former comprises at least one aromatic groups.

**12.** The method according to claim 11, wherein the radiation activatable radical former is an arene.

**13.** The method according to any of claims 11, wherein said radical former is selected from the group consisting of dibenzoyl peroxide, 3-benzoyl benzoic acid, 1,3-diibenzoyl propane and tri-fluoromethyl phenylketone.

**14.** The method according to any of claims 9 to 13, wherein said polymer chain segments are additionally covalently bound to each other through the reaction product of a cross-linker.

**15.** The method according to any of claims 9 to 14, wherein said electromagnetic radiation is UV radiation.

**16.** The method according to any of claims 9 to 15, wherein said method is carried out at a temperature below 80°C.

**17.** The method according to any of claims 9 to 16, wherein said polymer and said radiation activatable radical former are exposed to electromagnetic radiation for between 0.1 sec. and 30 min.

**18.** The method according to any of claims 9 to 17, wherein said method comprises the additional step of

d) regenerating the radical former after step c) of claim 9.

**19.** An absorbent article comprising a substantially liquid pervious topsheet, a substantially liquid impervious backsheet and an absorbent core between said topsheet and said backsheet, wherein said absorbent article comprises the superabsorbent polymer according to any one of claims 1 to 8.

**20.** An absorbent article comprising superabsorbent polymers, said superabsorbent polymers being made according to a process of any one of claims 9 to 18.

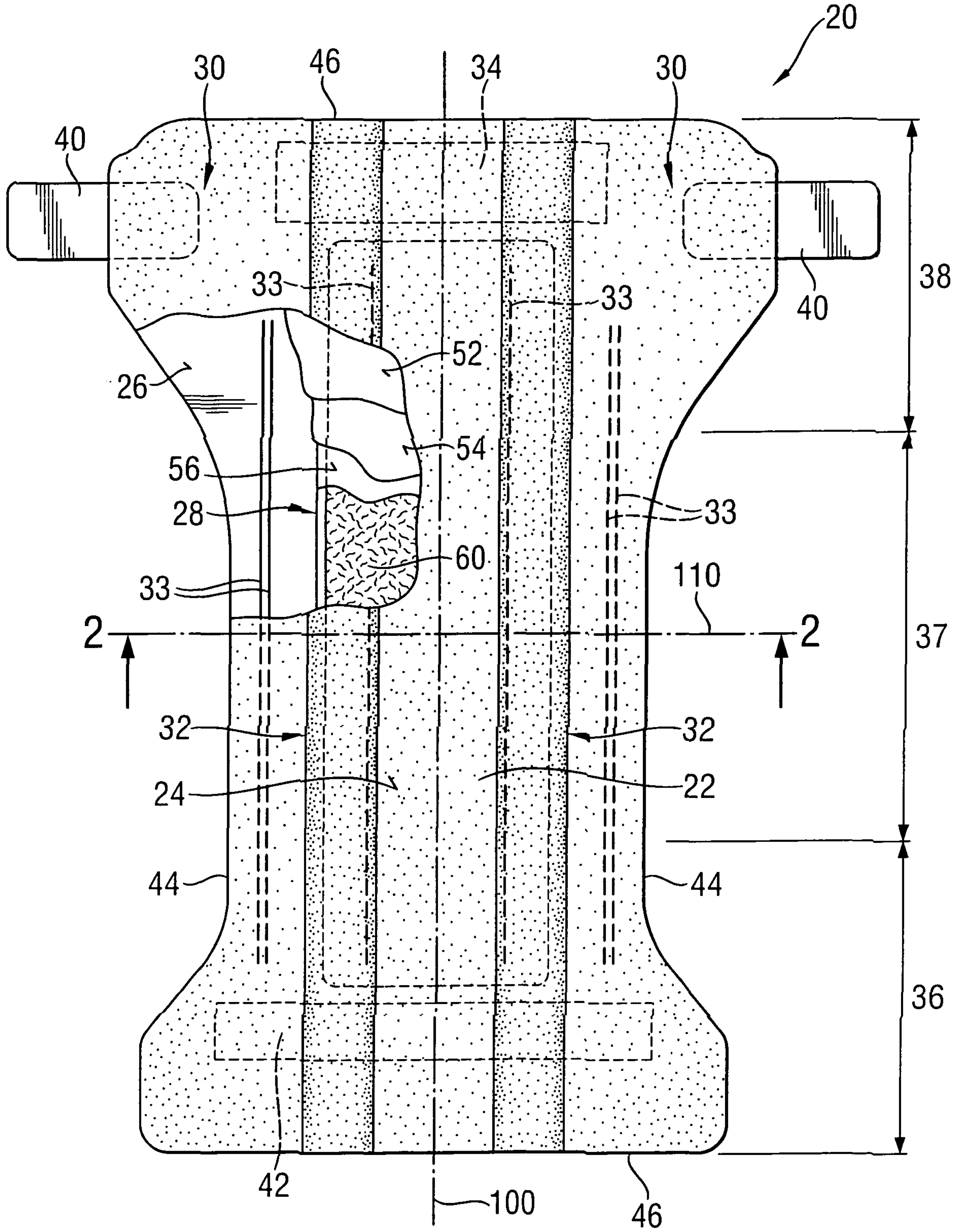
20
30
46
34
30
40
38
40
33
33
52
26
54
56
33
28
33
60
33
110
2
2
37
32
32
24
22
44
44
36
42
100
46

Fig. 1

*Fig. 2*

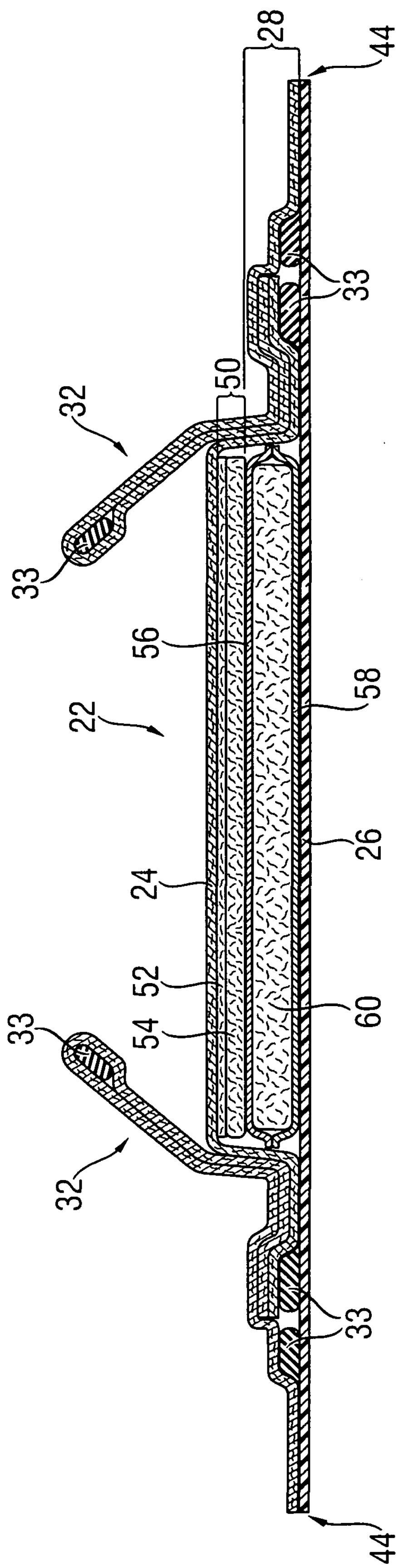

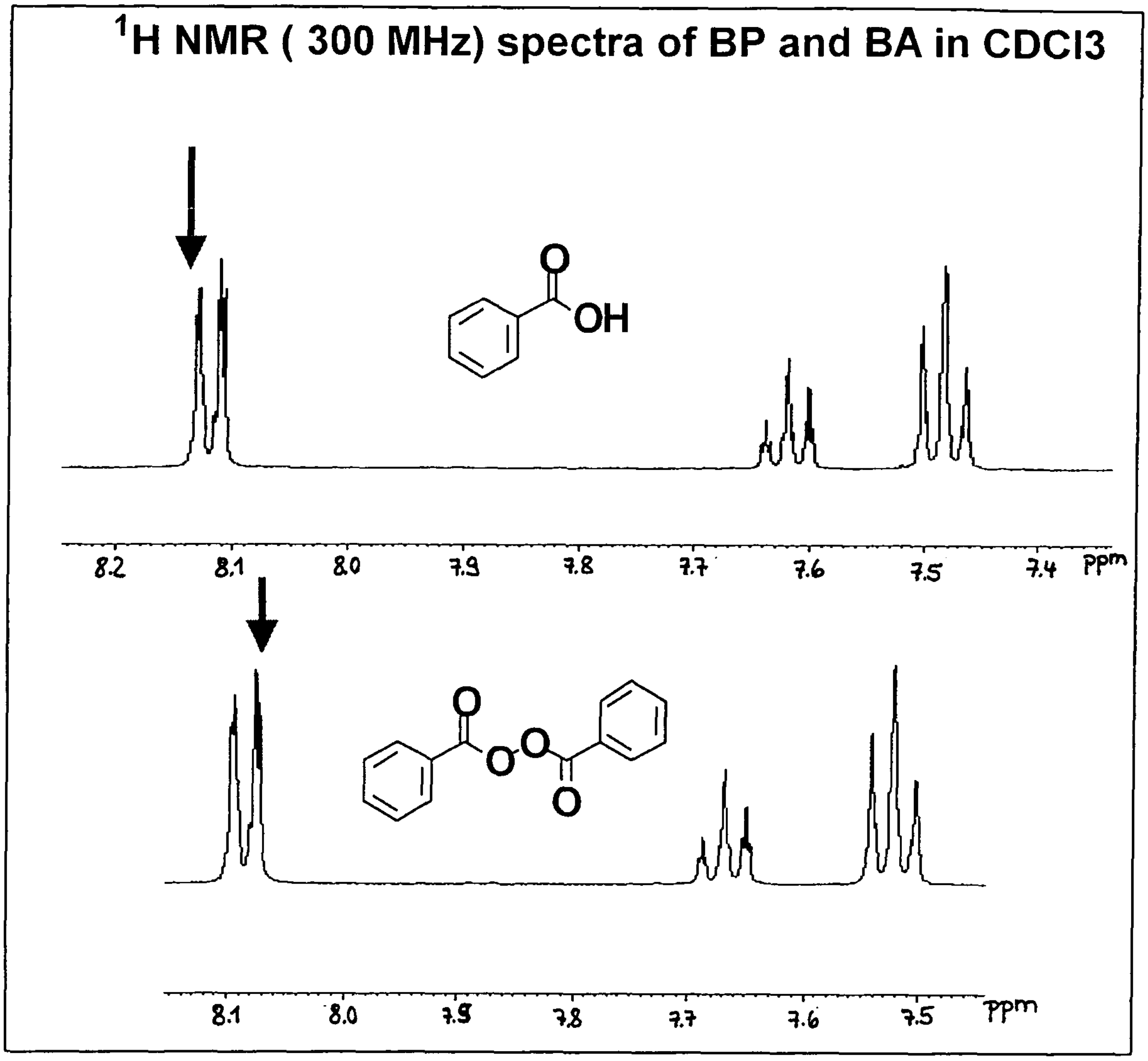

$^1$H NMR ( 300 MHz) spectra of BP and BA in CDCl3
8.2 8.1 8.0 7.9 7.8 7.7 7.6 7.5 7.4 ppm
8.1 8.0 7.9 7.8 7.7 7.6 7.5 ppm


Figure 3

## $^1$H-NMR (300MHz) Spectra of PAA + BP in $CDCl_3$

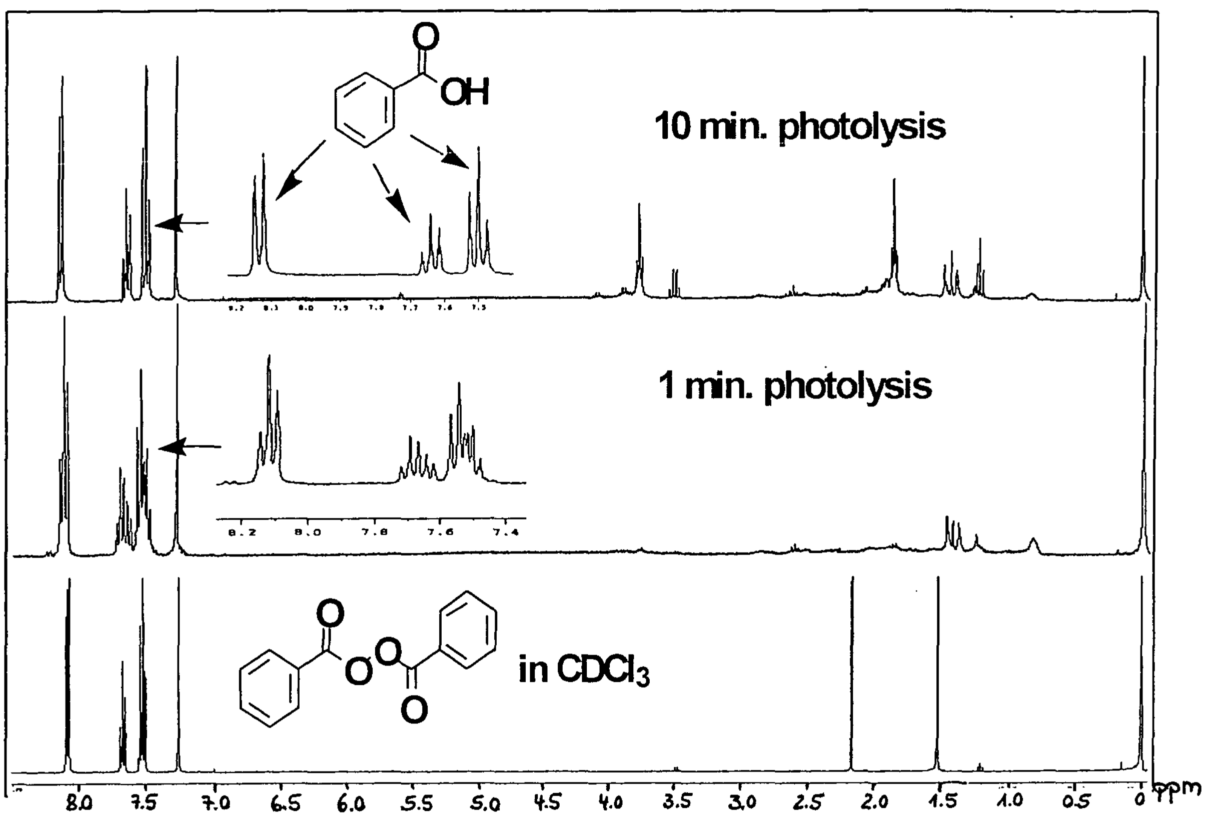


Figure 4

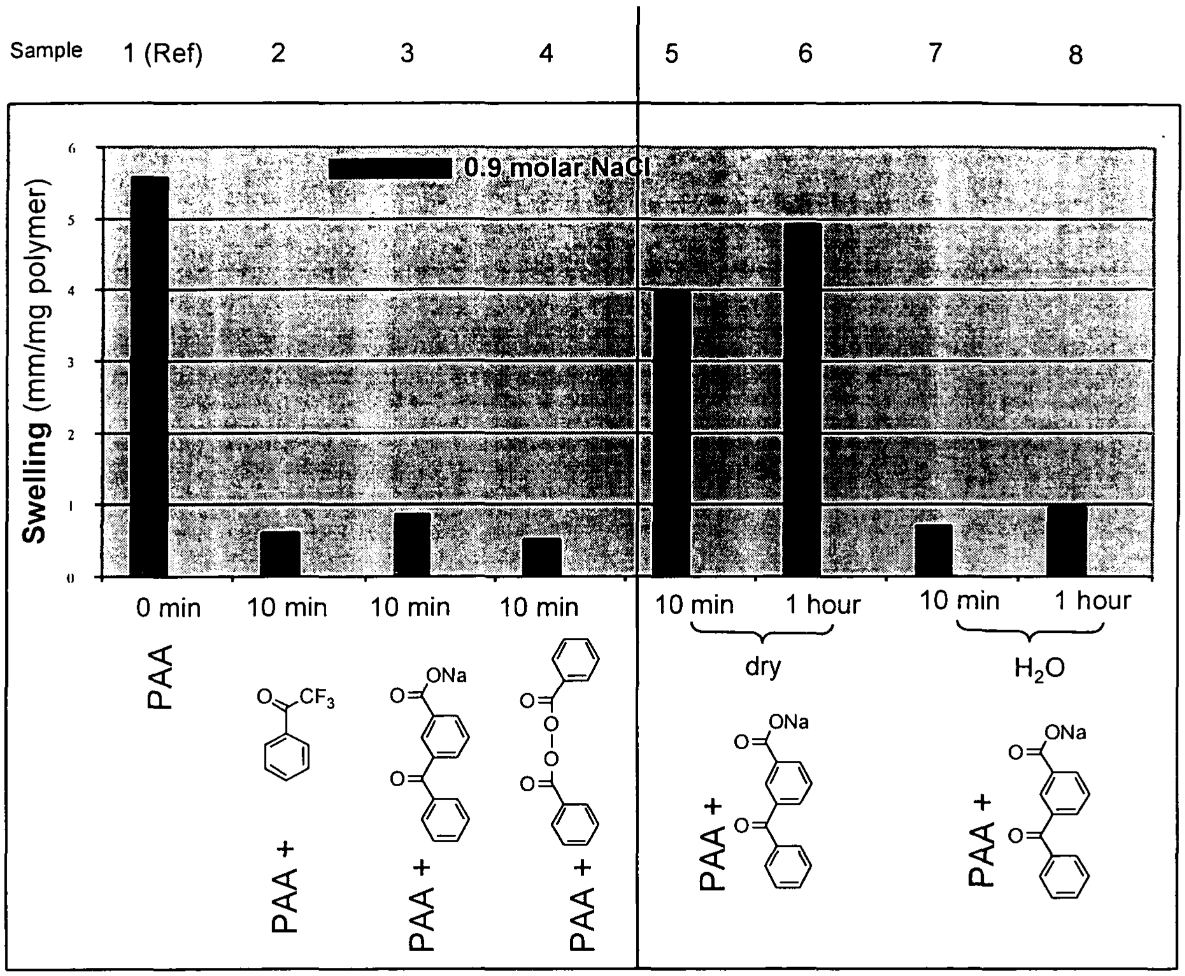
Sample
1 (Ref)
2
3
4
5
6
7
8
0.9 molar NaCl
Swelling (mm/mg polymer)
0 min
10 min
10 min
10 min
10 min
1 hour
dry
10 min
1 hour
H2O
PAA
PAA +
PAA +
PAA +
PAA +
PAA +

**Figure 5**

European Patent Office

# EUROPEAN SEARCH REPORT

Application Number
EP 04 00 4132

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 579 958 B2 (WILSON LARRY R)<br>17 June 2003 (2003-06-17)<br>* column 2, line 46 - column 3, line 31 *<br>* column 4, line 6 - line 30 *<br>* column 5, line 1 - line 51 *<br>* column 6, line 16 - line 28 *<br>----- | 1-4,6,19 | A61L15/60 |
| D,X | EP 0 509 708 A (DOW CHEMICAL CO)<br>21 October 1992 (1992-10-21)<br>* page 2, line 1 - line 33 *<br>* page 3, line 23 - line 51 *<br>* page 4, line 13 - line 45 *<br>* claims 1,5 *<br>----- | 1-4,6,8,<br>19 | |
| X | US 5 883 158 A (UMADA AKIRA ET AL)<br>16 March 1999 (1999-03-16)<br>* column 2, line 50 - column 3, line 35 *<br>* column 4, line 3 - line 26 *<br>* example 1 *<br>----- | 1-4,6,19 | |
| X | WO 99/55393 A (SCA HYGIENE PROD AB ;<br>STRANDQVIST KERSTI (SE))<br>4 November 1999 (1999-11-04)<br>* page 2, line 24 - line 28 *<br>* page 3, line 30 - page 4, line 13 *<br>* page 6, line 2 - line 3 *<br>* page 6, line 20 - line 31 *<br>* claims 1,2,4 *<br>----- | 1,19 | TECHNICAL FIELDS SEARCHED (Int.Cl.7)<br>A61L<br>C08F |
| X | DATABASE WPI<br>Section Ch, Week 199002<br>Derwent Publications Ltd., London, GB;<br>Class A14, AN 1990-011701<br>XP002293849<br>& JP 01 292103 A (MITSUBISHI RAYON CO LTD)<br>24 November 1989 (1989-11-24)<br>* abstract *<br>----- | 9-11,<br>15-18,20 | |

The present search report has been drawn up for all claims

| Place of search | Date of completion of the search | Examiner |
|---|---|---|
| The Hague | 31 August 2004 | Menidjel, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 04 00 4132

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2004

| Patent document cited in search report | Publication date | Patent family member(s) | Publication date |
|---|---|---|---|
| US 6579958 B2 | 14-11-2002 | US 2002169252 A1 | 14-11-2002 |
| | | CN 1402644 T | 12-03-2003 |
| | | EP 1244473 A1 | 02-10-2002 |
| | | JP 2003516431 T | 13-05-2003 |
| | | WO 0141818 A1 | 14-06-2001 |
| | | US 2002193492 A1 | 19-12-2002 |
| EP 0509708 A | 21-10-1992 | AT 161549 T | 15-01-1998 |
| | | AU 1487992 A | 22-10-1992 |
| | | CA 2066010 A1 | 16-10-1992 |
| | | DE 69223674 D1 | 05-02-1998 |
| | | DE 69223674 T2 | 09-07-1998 |
| | | DK 509708 T3 | 31-08-1998 |
| | | EP 0509708 A1 | 21-10-1992 |
| | | ES 2110470 T3 | 16-02-1998 |
| | | FI 921668 A | 16-10-1992 |
| | | JP 3283570 B2 | 20-05-2002 |
| | | JP 5156034 A | 22-06-1993 |
| | | KR 195778 B1 | 15-06-1999 |
| | | MX 9201747 A1 | 01-08-1993 |
| | | NO 921492 A | 16-10-1992 |
| | | US 5633316 A | 27-05-1997 |
| US 5883158 A | 16-03-1999 | JP 3118779 B2 | 18-12-2000 |
| | | JP 8053550 A | 27-02-1996 |
| | | JP 2938775 B2 | 25-08-1999 |
| | | JP 8176311 A | 09-07-1996 |
| | | CN 1161047 A ,B | 01-10-1997 |
| | | DE 69505398 D1 | 19-11-1998 |
| | | DE 69505398 T2 | 11-03-1999 |
| | | EP 0775161 A1 | 28-05-1997 |
| | | ES 2123278 T3 | 01-01-1999 |
| | | WO 9605234 A1 | 22-02-1996 |
| WO 9955393 A | 04-11-1999 | SE 511857 C2 | 06-12-1999 |
| | | AU 758225 B2 | 20-03-2003 |
| | | AU 4298899 A | 16-11-1999 |
| | | CA 2330008 A1 | 04-11-1999 |
| | | EP 1073482 A1 | 07-02-2001 |
| | | JP 2002513043 T | 08-05-2002 |
| | | PL 344082 A1 | 24-09-2001 |
| | | RU 2216359 C2 | 20-11-2003 |
| | | SE 9801490 A | 29-10-1999 |
| | | WO 9955393 A1 | 04-11-1999 |
| | | TW 457102 B | 01-10-2001 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 04 00 4132

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2004

| Patent document cited in search report | Publication date | Patent family member(s) | Publication date |
|---|---|---|---|
| JP 1292103 A | 24-11-1989 | NONE | |

EPO FORM P0459

For more details about this annex : see Official Journal of the European Patent Office, No. 12/82